(19) **Europäisches Patentamt / European Patent Office / Office européen des brevets**

(11) **EP 4 303 339 A1**

(12) **EUROPEAN PATENT APPLICATION**
published in accordance with Art. 153(4) EPC

(43) Date of publication:
**10.01.2024 Bulletin 2024/02**

(21) Application number: **22762667.8**

(22) Date of filing: **04.03.2022**

(51) International Patent Classification (IPC):
***C23C 16/48*** (2006.01)

(86) International application number:
**PCT/ES2022/070121**

(87) International publication number:
**WO 2022/184963 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.03.2021 ES 202130191**

(71) Applicant: **Universidad de Vigo**
**36310 Vigo (Pontevedra) (ES)**

(72) Inventors:
• **POU ÁLVAREZ, Pablo**
 **36280 Vigo (ES)**

• **RIVEIRO RODRÍGUEZ, Antonio**
 **36280 Vigo (ES)**
• **DEL VAL GARCÍA, Jesús**
 **36280 Vigo (ES)**
• **COMESAÑA PIÑEIRO, Rafael**
 **36280 Vigo (ES)**
• **BOUTINGUIZA LAROSI, Mohamed**
 **36280 Vigo (ES)**
• **LUSQUIÑOS RODRÍGUEZ, Fernando**
 **36280 Vigo (ES)**
• **POU SARACHO, Juan María**
 **36280 Vigo (ES)**

(74) Representative: **ABG Intellectual Property Law, S.L.**
**Avenida de Burgos, 16D**
**Edificio Euromor**
**28036 Madrid (ES)**

(54) **METHOD FOR CONTROLLED CORROSION GUIDANCE ON A MATERIAL OR ARTICLE TO BE TREATED BY MEANS OF A LASER BEAM**

(57) The present invention discloses a method for the controlled guidance of corrosion by the action of a laser beam. This method enables the selective adjustment of the speed of corrosion in each area of a target component and, therefore, the driving of the corrosion towards the areas of interest. With the application of this method, the corrosion process is guided by the laser treatment and becomes predictable and controllable. The method is applicable in all those sectors of activity where it is necessary to control corrosion.

EP 4 303 339 A1

## Description

## TECHNICAL FIELD

The present invention relates to techniques for protecting materials against corrosion. In particular, it relates to a method for the controlled guidance of corrosion on a material or article to be treated by the action of a laser beam.

The technique described in the present invention enables guiding corrosion in the desired direction in metallic materials or articles by the action of a laser beam.

## BACKGROUND OF THE INVENTION

Corrosion is a problem that affects many engineering fields, as it represents the loss of functionality of corroded materials or components, giving rise to serious personal, environmental, and economic risks.

Corrosion occurs as a result of a chemical etching sustained by the material due to its environment. Although any material is susceptible to sustaining corrosion, the most common cases of corrosion consist of the chemical etching of metals, which occurs mainly due to electrochemical etching, as metals have free electrons which are capable of forming electrochemical cells inside same. Electrochemical reactions require a conductive electrolyte, the medium for which is typically water. The corrosion process depends on a wide range of factors, including the materials and environmental properties. The speed at which corrosion takes place will depend to a certain extent on the temperature and the concentration of the reagents and the products. Other factors, such as mechanical stress and erosion can also contribute to accelerating the deterioration caused by corrosion.

Corrosion often develops randomly and unpredictably. Therefore, corrosion control is an extremely complex task. As a result, corrosion is generally seen as a problem to be prevented. To that end, preventive maintenance measures and the oversizing of components are usually applied despite the high economic costs associated with these strategies.

However, certain applications benefit from corrosion processes, such as batteries or biodegradable implants. In these cases, corrosion should not be prevented, but rather should proceed in a controlled manner. The local adjustment of the speed of corrosion in certain areas of a material, component or article to be treated would make it possible to regulate specific chemical reactions or the design of degradable components with a customized mechanical performance.

The ways to achieve that type of precise control of the corrosion process are very limited. On one hand, the local adjustment of the environmental properties seems at least difficult due to the stirring and/or diffusion processes, if not impossible due to restrictions in the specific application.

The alternative option is to adapt the properties of the material, component, or article to be treated itself. The precise adaptation of the properties of said material may be unfeasible from the perspective of manufacturing processes and may compromise the mechanical, thermal, and/or electrical performance of the material in question. Therefore, the control of the properties of the surface of the material seems to be the most reasonable approach.

The application of different types of (metallic, ceramic, organic) coatings is, therefore, usually the preferred surface modification technique when preventing corrosion. A characteristic that is common to all coatings is that the new layer is applied globally along the entire component to be protected. Therefore, a local adjustment of the corrosion properties cannot be achieved by means of these methods.

As an alternative, the laser-based surface treatment techniques enable the control of key surface properties that govern corrosion phenomena, such as the composition, microstructure, topography, or residual mechanical stresses. Furthermore, by adapting the processing conditions, a high degree of precision is achieved, even on a nanometric scale, making a local adjustment of these properties possible.

The present invention discloses a method for the controlled guidance of corrosion by the action of a laser beam.

## SUMMARY OF THE INVENTION

The present invention provides a method for the controlled guidance of corrosion on a material or article to be treated according to claim 1, the use of said method for the manufacture of resorbable metallic osteosynthesis plates that can be reabsorbed by the human body according to claim 9, and the use of said method for the manufacture of anodes for the cathodic protection of steel reinforcements in constructions made of concrete according to claim 10. The dependent claims define preferred embodiments of the invention.

The first inventive aspect relates to a method for the controlled guidance of corrosion on a material or article to be treated comprising the following steps:

- providing a guidance strategy for guiding corrosion on the material or article to be treated, such that a map with guidance directions for guiding corrosion is defined;

- establishing at least one laser beam surface treatment on the material or article to be treated by means of an increasing exposure, providing different speeds of corrosion in the material or article;

- applying at least one laser beam surface treatment on at least one area of the material or article to be treated with an increasing exposure in the direction indicated by the aforementioned map with guidance directions.

In the context of the invention, the material or article to be treated is the material, composite, or article on which the different steps of the method of the invention will be carried out.

Increasing exposure of the material or article to be treated to the laser beam, or simply "exposure", shall be understood to mean the amount of energy that the action of the laser beam provides to the article on a certain surface and in a certain period of time, measured as a ratio between the power of the laser and the processing speed.

Corrosion shall be understood to mean the phenomenon of the destruction of a material due to chemical etching.

The step of providing at least one laser beam surface treatment on the material or article to be treated consists of a modification of the surface of said material by the action of the laser beam, such that the speed of corrosion of the treated area of the surface of the material decreases as compared with the speed of corrosion of the untreated material. This modification may be due to different mechanisms: surface remelting, grain size modification, surface texture modification, surface wettability modification, surface alloying element evaporation, surface material ablation, or by the set of all or several of these mechanisms.

Said step of providing at least one laser beam surface treatment on the material or article to be treated is preceded by a prior study of the speed of corrosion of the untreated material. The reduction in the speed of corrosion achieved by means of treatments performed at different values of exposure to the applied laser beam is subsequently studied. Different treatments on a certain material which give rise to decreasing speeds of corrosion are thereby defined and ordered. Therefore, the step of applying the surface treatment begins with a lower exposure treatment and gives rise to a higher speed of corrosion, and higher exposure treatments are applied when needed.

In one embodiment, the method comprises performing a plurality or a set of laser beam surface treatments to be performed on the material or article to be treated, which are performed with an increase in the exposure in defined stages. In another embodiment, at least one laser beam surface treatment on the material or article to be treated is performed by means of a gradual increase in exposure. In both embodiments, the initial value of the exposure can be zero. These embodiments enable the adaptation of the speeds of corrosion of different parts of the material or article to be treated, depending on the final application that is sought with the controlled guidance of corrosion.

Laser surface treatments on the material or article to be treated by means of an increasing exposure must consist of at least one treatment in at least one area of the material or article to be treated with a value of exposure to the laser beam that provides a higher speed of corrosion than in the untreated area or areas of the material or article, and applied according to a defined strategy, which ultimately enables the guidance of corrosion.

In one embodiment, the laser surface treatments on the material or article to be treated by means of an increasing exposure consist of at least two with different values of exposure to the laser beam (including the value zero) which give rise to at least two different speeds of corrosion in the material or article.

In one embodiment of the method object of the present invention, the material or article to be treated is a metal, a ceramic, a polymer, natural rock, a hybrid material (also known as "composite"), etc. In a particular embodiment, the material or article to be treated is a lithium, magnesium, titanium, manganese, niobium, thallium, vanadium, zinc, chromium, cadmium, indium, gallium, iron, cobalt, copper, nickel, silver, or tin alloy, or a combination of all or several of these alloys.

In one embodiment of the method of the present invention, four types of treatments with different exposure to the laser beam which give rise to different speeds of corrosion are established. The orderly combination of these four types of treatment (from lower to higher exposure) give rise to a guidance of the corrosion on the material to be treated.

In one embodiment of the method of the present invention, two types of treatments with different exposure to the laser beam which give rise to different speeds of corrosion are established. The orderly combination of these two types of treatment (from lower to higher exposure) give rise to a guidance of the corrosion on the material to be treated.

In one embodiment of the method object of the present invention, in the step of establishing a set of laser surface treatments on the material or article, said laser beam comes from a laser source the wavelength of which is within the range of 100 nm to 11000 nm. Advantageously, this range of wavelengths enables the handling of the radiation laser without the need to use means of protection against ionizing radiation.

In one embodiment of the method, the mean power of the laser source is within the range of 1 W to 5000 W. Advantageously, this range of power of the laser source enables obtaining treatments that modify the speed of corrosion in a large number of materials with different chemical compositions.

In one embodiment, the laser source used to generate laser beam radiation is selected from Nd:YAG, Nd:glass, Nd:YVO$_4$, Er:YAG, Yb:YAG, Tm:YAG, diode, fiber, disk, CO$_2$, CO, HeCd, copper vapor, iodine, argon, krypton lasers or chemical lasers (HF, DF). Advantageously, the use of laser sources of this type enables obtaining treatments that modify the speed of corrosion in a large number of materials with different chemical compositions.

In one embodiment, the laser source used emits radiation in pulsed or continuous mode. In a more particular embodiment, the laser used emits in pulsed mode with a pulse duration between milliseconds and femtoseconds and more favorably in the range of 1 to 500 nanoseconds. Advantageously, the use of this range of laser pulse duration enables obtaining treatments that modify the speed of corrosion in a large number of materials with different chemical compositions.

In one embodiment of the method, the material or article to be treated has a non-planar shape and the laser scans the surface of the material or article to be treated by means of a three-dimensional optical scanning system.

In one embodiment of the method, the material or article to be treated has a planar shape and the laser beam scans the surface of the material or article to be treated by means of a two-dimensional optical scanning system.

Optionally, the method of controlled corrosion guidance by means of laser object of the present invention is carried out in a vacuum or in the presence of an oxidizing gas atmosphere. In one embodiment, this oxidizing gas can be $O_2$, $CO_2$, or mixtures thereof.

Advantageously, this oxidizing gas atmosphere promotes the formation of oxides during the action of the laser beam on the material such that the speed of corrosion thereof is modified.

The method of the present invention does not require the application of paints, coatings, protective layers, the addition of alloying elements, or any other type of additive, to the material object of the treatment. The results are obtained with a relatively simple system based on the use of a single laser beam on the material or article to be treated without the need for prior machining or preconditioning of its surface.

Furthermore, the method enables the selective adjustment of the speed of corrosion in each area of a target material or article and, therefore, the driving of the corrosion towards the regions or areas of interest. With the application of this method, the corrosion process is guided by the laser treatment and becomes predictable and controllable.

A second inventive aspect relates to the use of the method of the first inventive aspect for the manufacture of a resorbable metallic osteosynthesis plate that can be reabsorbed by the human body.

By applying the method of the present invention, resorbable metallic osteosynthesis plates for fracture fixation in orthopedics, neurosurgery, maxillofacial surgery, etc., can be manufactured. Current metallic osteosynthesis plates are not resorbable, whereas the currently available resorbable plates are made of polymeric materials with low mechanical properties. By means of the method object of the present invention, osteosynthesis plates based on a magnesium alloy which is resorbable by the human body, but which maintains the material properties necessary to withstand the mechanical stresses during the time necessary for bone regeneration, can be manufactured.

A third inventive aspect relates to the use of the method of the first inventive aspect for the manufacture of an anode for the cathodic protection of steel reinforcements in constructions made of concrete with guided and controlled corrosion, having a prolonged duration and protection.

By applying the method object of the present invention to a protection anode, its degradation can be guided and controlled, increasing the service life of the anode itself and extending the time of the protection. The effects of the self-corrosion can be prevented, increasing the amount of anodic material available for corrosion when cathodic protection is required. The result is a longer service life of the constructions made of reinforced concrete.

## DESCRIPTION OF THE DRAWINGS

To complement the description that is being made and for the purpose of helping to better understand the features of the invention, the following figures are attached as an integral part of said description.

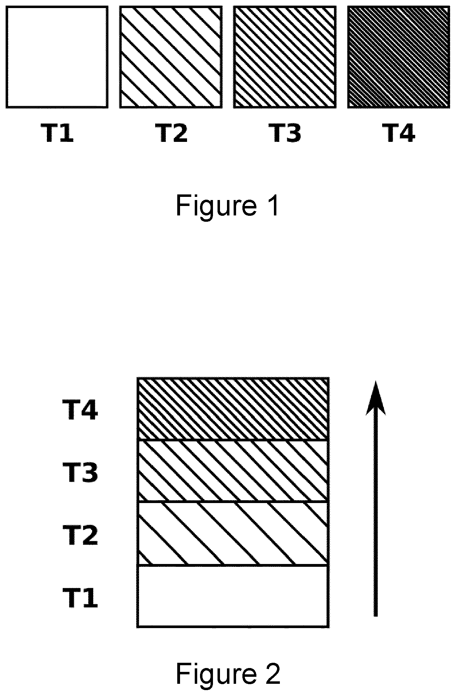

- Figure 1: this figure schematically depicts four types of treatments performed on a material or article to be treated according to one embodiment of the invention.

- Figure 2: this figure shows a guidance strategy for guiding corrosion on a material or article to be treated according to one embodiment of the invention, such that a direction for guiding the corrosion following the direction of the arrow is defined.

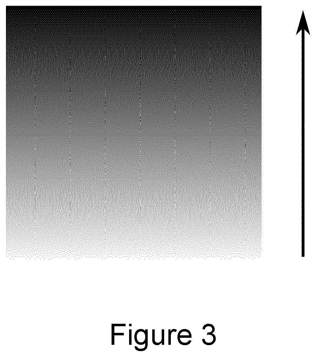

- Figure 3: this figure shows a guidance strategy for guiding corrosion on a material or article to be treated according to another embodiment of the invention, such that a direction for guiding the corrosion following the direction of the arrow is defined.

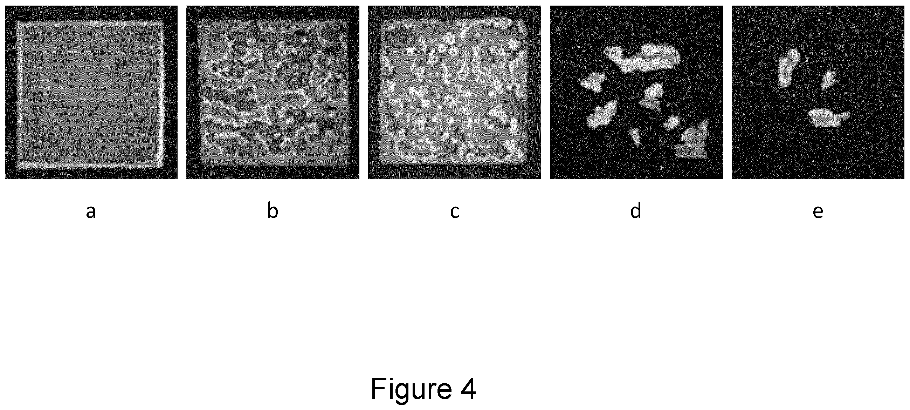

- Figures 4a to 4e: these figures show the uncontrolled temporal evolution of a magnesium alloy plate subjected to corrosion in physiological saline solution, Figure 4a corresponding to the article before the start of the test and successive Figures 4b to 4e to the random evolution of the corrosion of the article over 3 weeks.

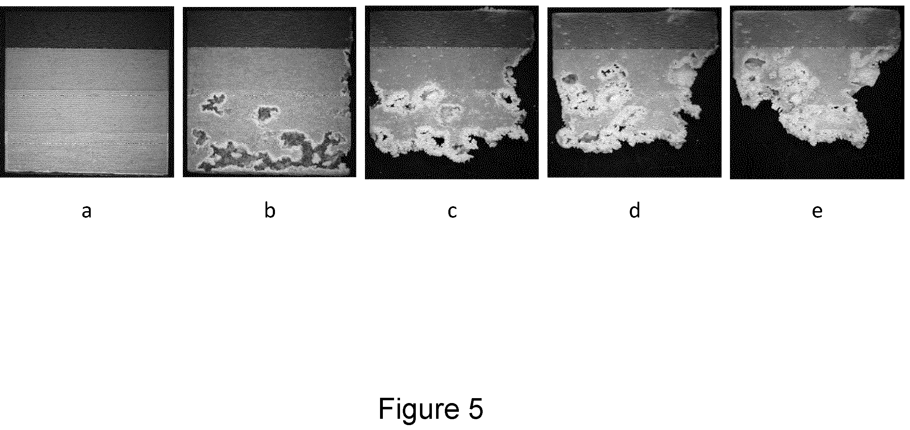

- Figures 5a to 5e: these figures show the controlled temporal evolution of a magnesium alloy plate subjected to corrosion in physiological saline solution to which the guidance strategy according to the embodiment of Figure 2 has been applied. Figure 5a shows the article before the start of the corrosion test, whereas successive Figures 5b to 5e show the controlled or guided evolution of the same article over 3 weeks.

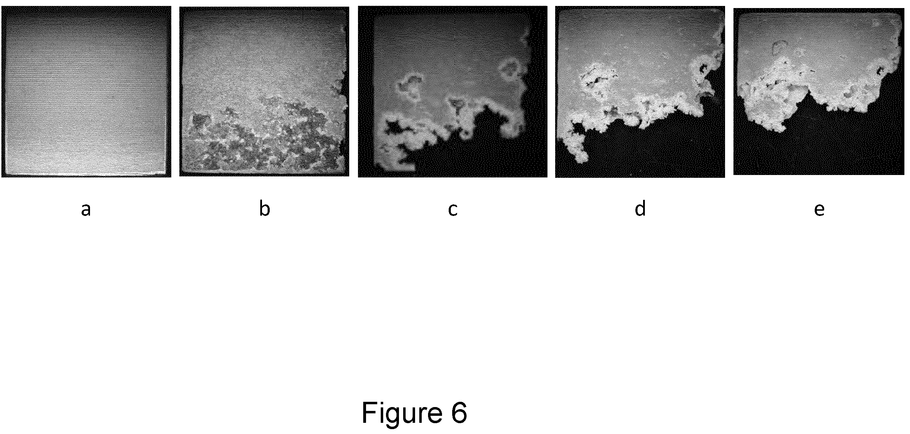

- Figures 6a to 6e: these figures show the controlled temporal evolution of a magnesium alloy plate subjected to corrosion in physiological saline solution to which the guidance strategy according to the embodiment of Figure 3 has been applied. Figure 6a shows the article before the start of the corrosion test, whereas successive Figures 6b to 6e show the controlled or guided evolution of the same article over 3 weeks.

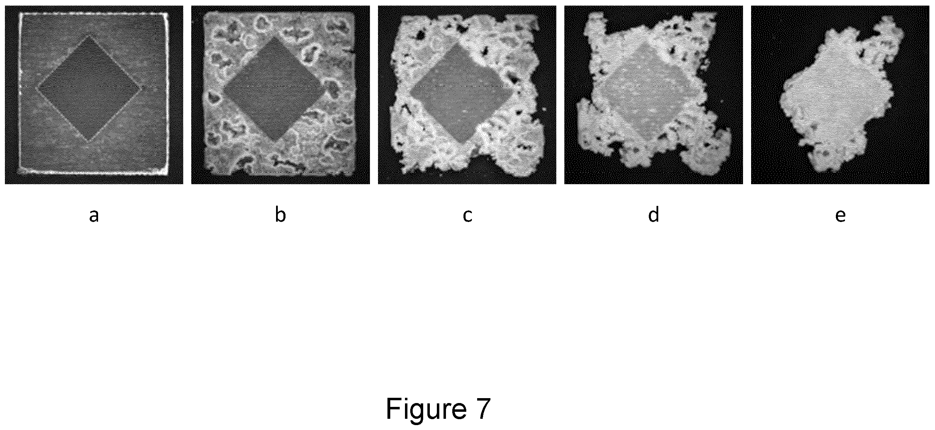

- Figures 7a to 7e: these figures show the controlled temporal evolution of a magnesium alloy plate subjected to corrosion in physiological saline solution to which a guidance strategy according to another embodiment of the invention in which the central diamond shape has been treated with high exposure (equivalent to treatment T4 of the embodiment of Figure 1) and the rest of the article has been treated with low exposure (equivalent to treatment T1 of the embodiment of Figure 1) has been applied. Figure 7a shows the article before the start of the corrosion test, whereas successive Figures 7b to 7e show the controlled or guided evolution of the same article over 3 weeks.

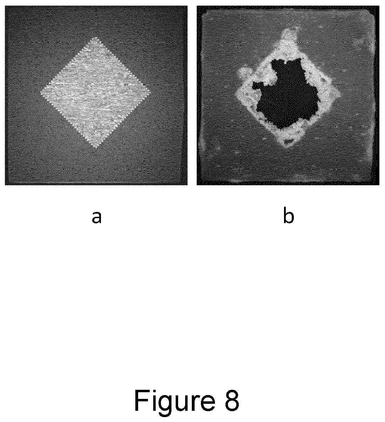

- Figures 8a to 8b: these figures show the controlled temporal evolution of a magnesium alloy plate subjected to corrosion in physiological saline solution to which a guidance strategy according to another embodiment of the invention in which the central diamond shape has been treated with low exposure (equivalent to treatment T1 of the embodiment of Figure 1) and the rest of the article has been treated with high exposure (equivalent to treatment T4 of the embodiment of Figure 1) has been applied. Figure 8a shows the article before the start of the corrosion test, whereas Figure 8b shows the state of the same article after 3 weeks.

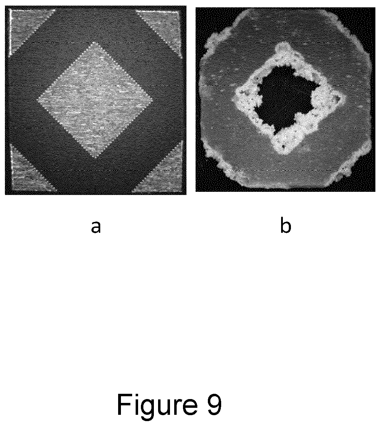

- Figures 9a to 9b: these figures show the controlled temporal evolution of a magnesium alloy plate subjected to corrosion in physiological saline solution to which a guidance strategy according to another embodiment of the invention in which the central diamond shape and the triangles of the four corners of the article have been treated with low exposure (equivalent to treatment T1 of the embodiment of Figure 1) and the rest of the article has been treated with high exposure (equivalent to treatment T4 of the embodiment of Figure 1) has been applied. Figure 9a shows the article before the start of the corrosion test, whereas Figure 9b shows the state of the same article after 3 weeks.

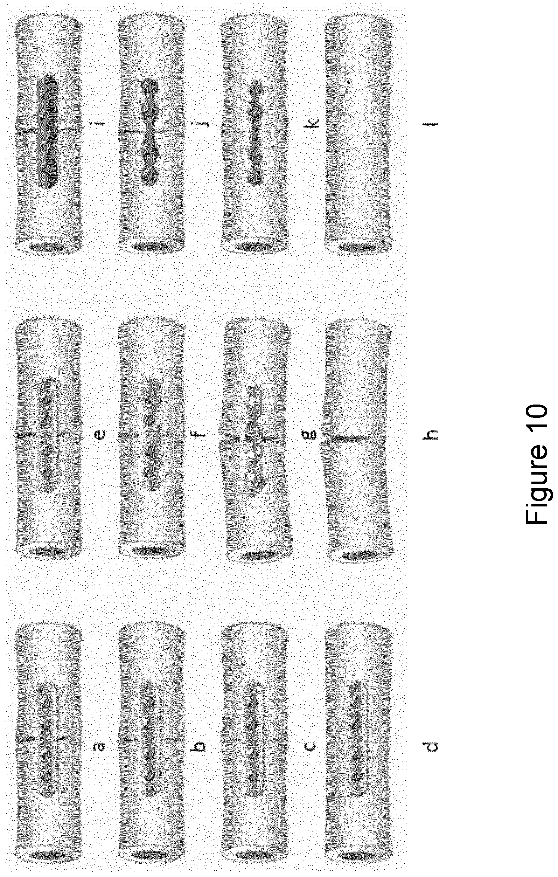

- Figures 10a to 10l: these figures show the application of the method object of the invention according to a particular embodiment to the manufacture of biodegradable osteosynthesis plates.

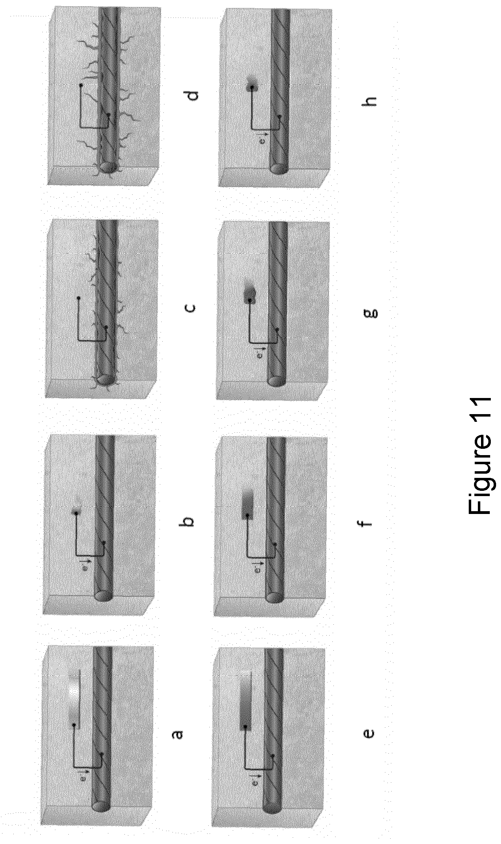

- Figures 11a to 11h: these figures show the application of the method object of the invention according to a particular embodiment for the manufacture of anodes for the cathodic protection of steel reinforcements in constructions made of concrete.

## DETAILED DISCLOSURE OF THE INVENTION

The present invention relates to a method for the controlled guidance of corrosion on a material or article to be treated.

Figure 1 schematically depicts four types of treatment performed by means of a laser beam on a material or article to be treated. The difference between the treatments lies in the different exposure. In that sense, the exposure in treatment T1 is lower than in treatment T2, the exposure in treatment T2 is lower than in treatment T3, and the exposure in treatment T3 is lower than in treatment T4. Therefore, the speed of corrosion of a material subjected to treatment T1 is higher than the speed of corrosion of the same material subjected to treatment T2. In turn, the speed of corrosion of this material subjected to treatment T2 is higher than the speed of corrosion of the same material subjected to treatment T3, and the speed of corrosion of this material subjected to treatment T3 is higher than the speed of corrosion of the same material subjected to treatment T4.

Figure 2 shows an exemplary guidance strategy for guiding corrosion on a material or article to be treated, such that a direction for guiding the corrosion following the direction of the arrow is defined. The four different areas correspond to laser treatments performed with different exposures on the surface of the material or article to be treated. Therefore, the exposure is an increasing exposure according to the following guideline: exposure in T1 is higher than the exposure in T2; the exposure in T2 is higher than the exposure in T3; and the exposure in T3 is higher than the exposure in T4.

Figure 3 shows an exemplary guidance strategy for guiding corrosion on a material or article to be treated, such that a direction for guiding the corrosion following the direction of the arrow is defined. In this case, the exposure of the laser beam on the material or article to be treated increases gradually in the direction of the arrow.

Figure 4 shows the temporal evolution of an AZ31 magnesium alloy plate subjected to corrosion in physiological saline solution (normal saline solution, containing 9 grams per liter of ClNa). Figure 4a shows the article before the start of the corrosion test, whereas successive Figures 4b, 4c, 4d, and 4e show the evolution of the same article over 3 weeks. It can be seen that corrosion occurs in a random, unpredictable manner, causing aggression on the article in all directions. After 3 weeks, the article is completely destroyed.

Figure 5 shows the temporal evolution of an AZ31 magnesium alloy plate subjected to corrosion in physiological saline solution (normal saline solution, containing 9 grams per liter of ClNa), to which the method of controlled corrosion guidance object of the present invention has been applied. This case follows the strategy shown in Figure 2. Figure 5a shows the article before the start of the corrosion test, whereas successive Figures 5b, 5c, 5d, and 5e show the evolution of the same article over 3 weeks. It can be seen that corrosion occurs in a guided or controlled manner, starting at the lower part of the article and progressing towards the upper part, following the direction established by the method of guiding object of the present invention. After 3 weeks, the article has not corroded in its entirety.

Figure 6 shows the temporal evolution of a magnesium alloy plate subjected to corrosion in physiological saline solution (normal saline solution, containing 9 grams per liter of ClNa), to which the method of controlled corrosion guidance object of the present invention has been applied. This case follows the strategy shown in Figure 3. Figure 6a shows the article before the start of the corrosion test, whereas successive Figures 6b, 6c, 6d, and 6e show the evolution of the same article over 3 weeks. It can be seen that corrosion occurs in a guided or controlled manner, starting at the lower part of the article and progressing towards the upper part, following the direction established by the method of guiding object of the present invention. After 3 weeks, corrosion reached only a little more than half of the article.

Figure 7 shows the temporal evolution of a magnesium alloy plate subjected to corrosion in physiological saline solution (normal saline solution, containing 9 grams per liter of ClNa), to which the method of controlled corrosion guidance object of the present invention has been applied. In this case, the central diamond shape has been treated with high exposure (equivalent to treatment T4 of the embodiment of Figure 1) and the rest of the article has been treated with low exposure (equivalent to treatment T1 of the embodiment of Figure 1). Figure 7a shows the article before the start of the corrosion test, whereas successive Figures 7b, 7c, 7d, and 7e show the evolution of the same article over 3 weeks. It can be seen that corrosion occurs in a guided or controlled manner, starting at the periphery of the article and progressing towards the center thereof. After 3 weeks, the central diamond shape has not been corroded.

Figure 8 shows the temporal evolution of a magnesium alloy plate subjected to corrosion in physiological saline solution (normal saline solution, containing 9 grams per liter of ClNa), to which the method of controlled corrosion guidance object of the present invention has been applied. In this case, the central diamond shape has been treated with low exposure (equivalent to treatment T1 of the embodiment of Figure 1) and the rest of the article has been treated with high exposure (equivalent to treatment T4 of the embodiment of Figure 1). Figure 8a shows the article before the start of the corrosion test, whereas Figure 8b shows the state of the same article after 3 weeks. It can be seen that corrosion occurs in a guided or controlled manner, starting in the interior of the article and progressing towards the periphery thereof. After 3 weeks, corrosion has only affected the central diamond shape.

Figure 9 shows the temporal evolution of a magnesium alloy plate subjected to corrosion in physiological saline solution (normal saline solution, containing 9 grams per liter of ClNa), to which the method of controlled corrosion guidance object of the present invention has been applied. In this case, the central diamond shape and the triangles of the four corners of the article have been treated with low exposure (equivalent to treatment T1 of the embodiment of Figure 1) and the rest of the article has been treated with high exposure (equivalent to treatment T4 of the embodiment of Figure 1). Figure 9a shows the article before the start of the corrosion test, whereas Figure 9b shows the state of the same article after 3 weeks. It can be seen that corrosion occurs in a guided or controlled manner, starting in the interior and in the four corners of the article and progressing towards the areas treated with treatment type T4. After 3 weeks, corrosion has only affected the four corners and the central diamond shape.

Figure 10 shows the application of the method object of the invention to the manufacture of biodegradable osteosynthesis plates. Figures 10a, 10b, 10c, and 10d show the evolution in the regeneration of a bone fracture by means of a non-resorbable metallic osteosynthesis plate. When the bone has already been regenerated (Figure 10d), the osteosynthesis plate is still intact and a second surgical operation is required to return the patient to his or her initial condition, i.e., with the regenerated bone but without a metal plate inside his or her body. Figures 10e, 10f, 10g, and 10h show the same process as in the previous figures, but now using a plate made of a resorbable material. This material loses its mechanical integrity before complete bone regeneration (Figure 10f), so the bone is not properly regenerated (Figure 10h). Figures 10i, 10j, 10k, and 10l show the same process as in the two previous cases, but now using an osteosynthesis plate made of a resorbable magnesium alloy but subjected to the method of controlled corrosion guidance object of the present invention. In this case, corrosion occurs in a guided or controlled manner and at the same rate at which the bone regenerates (Figure 10j and Figure 10k). The result is complete bone regeneration and complete resorption of the osteosynthesis plate (Figure 10l). This means full recovery of bone functionality and avoiding a second operation to remove the osteosynthesis plate.

Figure 11 shows the application of the method object of the present invention for the manufacture of anodes for the cathodic protection of steel reinforcements in constructions made of concrete. Figures 11a, 11b, 11c, and 11d show the behavior of reinforced concrete when an anode is used for conventional cathodic protection. The anode undergoes an uncontrolled corrosion process. As a result, its service life is short and it ceases to protect the reinforcements from corrosion (Figure 11c), so a layer of corrosion products is formed on the reinforcements which strain and crack the concrete, causing its structural failure (Figure 11d).

When the anode for cathodic protection is subjected to the method object of the present invention, corrosion occurs in a guided or controlled manner, so the anode corrodes slowly (Figure 11g), whereby very notably increasing its service life and extending the time of duration of the cathodic protection (Figure 11h). The final result is a longer service life of the constructions made of reinforced concrete.

## EXAMPLES

*Example 1:*

To guide corrosion in an AZ31 magnesium alloy plate, a guidance strategy consisting of the corrosion having to start at the bottom part of the plate and progress towards the upper part thereof was established.

A set of laser surface treatments was established, with a Nd:YVO4 laser beam (1064 nm) with a rated power of 20 W being used, operating in pulsed mode with a 20-ns pulse duration and focused on the surface of an AZ31 magnesium alloy plate. With this laser and this material, four different treatments called T1, T2, T3, and T4 were established, and they gave rise to four different speeds of corrosion, with the speed of corrosion being higher in treatment T1 than in T2, the speed of corrosion being higher in T2 than in T3, and the speed of corrosion being higher in T3 than in T4.

The values of increasing exposure of the AZ31 magnesium alloy to the action of the laser beam, increasing exposure being understood to mean the Napierian logarithm of the quotient of the power of the laser squared, divided by the square root of the processing speed,

$$exposure \; = \; \ln\left(\frac{P^2}{\sqrt{v}}\right)$$

were the following for each treatment:

$$T1 = 3.3 \; W^2 \; m^{-0.5} \; s^{0.5},$$

$$T2 = 4.2 \; W^2 \; m^{-0.5} \; s^{0.5},$$

$$T3 = 5.1 \; W^2 \; m^{-0.5} \; s^{0.5},$$

and

$$T4 = 6.0 \; W^2 \; m^{-0.5} \; s^{0.5}.$$

A treatment was then performed with increasing exposure in stages as shown in Figure 2. Once the article made of AZ31 magnesium alloy was treated, a corrosion test in physiological saline solution was performed on an untreated AZ31 magnesium alloy plate (Figure 4) and the article made of magnesium alloy to which the method object of the invention that was just described in detail was applied (Figure 5).

As can be seen by comparing Figures 4 and 5, it can be seen that, in the untreated article, corrosion occurs in a random, unpredictable manner, causing aggression on the article in all directions (Figure 4). After 3 weeks, the article is completely destroyed.

However, in the article to which the method object of the present invention has been applied, according to the present embodiment, guidance of corrosion from top to bottom of the article, following the initially established direction (Figure 5), can be observed. After 3 weeks, the article has not corroded in its entirety.

*Example 2:*

To guide corrosion in an AZ31 magnesium alloy plate, a guidance strategy consisting of the corrosion having to start at the bottom part of the plate and progress towards the upper part thereof was established.

A set of laser surface treatments was established, with a Nd:YVO4 laser beam (1064 nm) with a rated power of 20 W being used, operating in pulsed mode with a 20-ns pulse duration and focused on the surface of an AZ31 magnesium alloy plate. With this laser and this material, different treatments which gave rise to different speeds of corrosion were established. These treatments successfully changed the value of exposure of the AZ31 magnesium alloy to the action of the laser beam.

The values of increasing exposure of the AZ31 magnesium alloy to the action of the laser beam, increasing exposure being understood to mean the Napierian logarithm of the quotient of the power of the laser squared, divided by the square root of the processing speed,

$$exposure \; = \; \ln\left(\frac{P^2}{\sqrt{v}}\right)$$

were gradually increased from a value of 2.2 $W^2$ $m^{-0.5}$ $s^{0.5}$ to a maximum value of 6.0 $W^2$ $m^{-0.5}$ $s^{0.5}$

A treatment was then performed with the gradually increasing exposure as shown in Figure 3. Once the article made of AZ31 magnesium alloy has been treated, a corrosion test in physiological saline solution was performed on an untreated AZ31 magnesium alloy plate (Figure 4) and the article made of magnesium alloy to which the method object of the invention that was just described in detail was applied (Figure 6).

As can be seen by comparing Figures 4 and 6, it can be seen that, in the untreated article, corrosion occurs in a random, unpredictable manner, causing aggression on the article in all directions (Figure 4). After 3 weeks, the article is completely destroyed.

However, in the article to which the method object of the present invention has been applied, according to the present embodiment, guidance of the corrosion from top to bottom of the article, following the initially established direction (Figure 6), can be observed. After 3 weeks, corrosion reached only a little more than half of the article.

*Example 3:*

To guide corrosion in an AZ31 magnesium alloy plate, a guidance strategy consisting of the corrosion having to start in the periphery of the article and progress towards the center thereof was established.

A set of laser surface treatments was established, with a Nd:YVO4 laser beam (1064 nm) with a rated power of 20 W being used, operating in pulsed mode with a 20-ns pulse duration and focused on the surface of an AZ31 magnesium alloy plate. With this laser and this material, two different treatments which gave rise to two different speeds of corrosion, with the speed of corrosion being higher in the first treatment than in the second treatment, were established.

The values of increasing exposure of the AZ31 magnesium alloy to the action of the laser beam, increasing exposure being understood to mean the Napierian logarithm of the quotient of the power of the laser squared, divided by the square root of the processing speed,

$$exposure \ = \ \ln\left(\frac{P^2}{\sqrt{v}}\right)$$

were 0 $W^2$ $m^{-0.5}$ $s^{0.5}$ for the first treatment and 6.0 $W^2$ $m^{-0.5}$ $s^{0.5}$ for the second treatment.

In this case, the central diamond shape was subjected to the second treatment and the rest of the article to the first treatment. Once the article made of AZ31 magnesium alloy has been treated, a corrosion test in physiological saline solution was performed on an untreated AZ31 magnesium alloy plate (Figure 4) and the article made of magnesium alloy to which the method object of the invention that was just described in detail was applied (Figure 7).

As can be seen by comparing Figures 4 and 7, it can be seen that, in the untreated article, corrosion occurs in a random, unpredictable manner, causing aggression on the article in all directions (Figure 4). After 3 weeks, the article is completely destroyed.

However, in the article to which the method object of the present invention has been applied, according to the present embodiment, guidance of the corrosion from the periphery towards the interior, following the initially established direction (Figure 7), can be observed. After 3 weeks, the central diamond shape has not been corroded.

*Example 4:*

This example is the result of applying the method object of the present invention according to the embodiment of Example 3, i.e., with the same material, with the same laser, and with the same values of increasing exposure as in said Example 3, but with the exception that in this Example 4, a guidance strategy for guiding corrosion such that corrosion must start at the center of the plate and progress towards the periphery is established.

It can be seen in Figure 8 that corrosion occurs in a guided manner, starting in the interior of the article and progressing towards the periphery thereof. After 3 weeks, corrosion has only affected the central diamond shape.

*Example 5:*

This example is the result of applying the method object of the present invention according to the embodiment of Example 3, i.e., with the same material, with the same laser, and with the same values of increasing exposure as in said Example 3, but with the exception that in this Example 5, a guidance strategy for guiding corrosion such that corrosion must be directed from the corners towards the center of the plate and from the center of the plate towards the corners is established.

It can be seen in Figure 9 that corrosion occurs in a guided manner, starting in the four corners and in the interior of the article and progressing towards the interior and towards the corners, respectively. After 3 weeks, corrosion has only affected the four corners and the central diamond shape.

Having sufficiently described the nature of the present invention, as well as the way of carrying it out to practice, it only remains to be added that it is possible to introduce in said invention, as a whole and in its constituent parts, changes in form, materials, and arrangement, provided that such alterations do not substantially change said invention.

**Claims**

1. Method for the controlled guidance of corrosion on a material or article to be treated comprising the following steps:

   - providing a guidance strategy for guiding corrosion on the material or article to be treated, said guidance strategy for guiding corrosion comprising a map with guidance directions for guiding corrosion;

- establishing at least one laser beam surface treatment to be applied on the material or article to be treated by means of an increasing exposure, providing at least two speeds of corrosion in the material or article to be treated;

- applying said at least one laser beam surface treatment on at least one area of the material or article to be treated with an increasing exposure in the direction indicated by the aforementioned map with guidance directions.

2. A method according to claim 1, wherein the increasing exposure corresponds to the formula

$$exposure \; = \; \ln\left(\frac{P^2}{\sqrt{v}}\right)$$

wherein

$P$ is the power of the laser, and
$v$ is the processing speed.

3. A method according to any of the preceding claims comprising a plurality of laser beam surface treatments performed with an increase in the exposure in defined stages, preferably two stages, and more preferably four stages.

4. A method according to any of claims 1 or 2, wherein the at least one surface treatment is performed by means of a gradual increase in exposure.

5. A method according to claim 1, wherein, in the step of establishing at least one laser beam surface treatment on the material or article to be treated, said laser beam comes from a laser source the wavelength of which is within the range of 100 nm to 11000 nm.

6. A method according to claim 1, wherein the mean power of the laser source is within the range of 1 W to 5000 W.

7. A method according to claim 1, wherein the laser source used to generate laser beam radiation is selected from Nd:YAG, Nd:glass, Nd:YVO$_4$, Er:YAG, Yb:YAG, Tm:YAG, diode, fiber, disk, CO$_2$, CO, HeCd, copper vapor, iodine, argon, krypton lasers or chemical lasers (HF, DF).

8. A method according to claim 1, wherein the laser source used emits radiation in pulsed or continuous mode.

9. A method according to any of claims 5 to 8, wherein the laser source used emits in pulsed mode with a pulse duration between milliseconds and femtoseconds and more favorably in the range of 1 to 500 nanoseconds.

10. A method according to claim 1, wherein the material or article to be treated has a non-planar shape and the laser beam scans the surface of the material or article to be treated by means of a three-dimensional optical scanning system.

11. A method according to claim 1, wherein the material or article to be treated has a planar shape and the laser beam scans the surface of the material or article to be treated by means of a two-dimensional optical scanning system.

12. A method according to claim 1, wherein the method is carried out in a vacuum or in the presence of an oxidizing gas atmosphere.

13. A method according to claim 12, wherein the oxidizing gas is O$_2$, CO$_2$, or mixtures thereof.

14. Use of the method according to the preceding claims for the manufacture of a resorbable metallic osteosynthesis plate that can be reabsorbed by the human body.

15. Use of the method according to claims 1 to 8 for the manufacture of an anode for the cathodic protection of steel reinforcements in constructions made of concrete with guided and controlled corrosion, having a prolonged duration and protection.

Figure 1

Figure 2

Figure 3

a          b          c          d          e

Figure 4

Figure 5

Figure 6

Figure 7

Figure 8

a        b

Figure 9

Figure 10

Figure 11

<table>
<tr><td colspan="2"><b>INTERNATIONAL SEARCH REPORT</b></td><td>International application No.<br>PCT/ES2022/070121</td></tr>
</table>

**A. CLASSIFICATION OF SUBJECT MATTER**

***C23C16/48*** (2006.01)

According to International Patent Classification (IPC) or to both national classification and IPC

**B. FIELDS SEARCHED**

Minimum documentation searched (classification system followed by classification symbols)
C23C

Documentation searched other than minimum documentation to the extent that such documents are included in the fields searched

Electronic data base consulted during the international search (name of data base and, where practicable, search terms used)

EPODOC, INVENES, WPI, XPESP

**C. DOCUMENTS CONSIDERED TO BE RELEVANT**

| Category* | Citation of document, with indication, where appropriate, of the relevant passages | Relevant to claim No. |
|---|---|---|
| A | KUMARI, R. et al.. Microstructure and corrosión behavior of laser induced periodic patterned titanium based alloy. Optics and Laser Technology, 27/03/2019, pages 196-213 [on line][retrieved the 21/09/2021]. Experimental method | 1-15 |
| A | COTTAM R.. Chapter 1. Environmental and Industrial Corrosion – Practical and Theoretical Aspects. Laser Materials Processing for Improved CorrosionPerformance, 12/12/2012, [on line] [retrieved the 21/09/2021]. Apartate 3.1 Laser Surface melting | 1-15 |
| A | MEZA PARIONA, M. et al.. Influence of laser surface treated on the characterization and corrosion behavior of Al–Fe aerospace alloys. Applied Surface Science, 2013, pages 76-85 [on line] [retrieved on 05/08/2021]. Conclusions | 1-15 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |
| Date of the actual completion of the international search<br>18/05/2022 | Date of mailing of the international search report<br>**(20/05/2022)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>B. Aragón Urueña<br><br>Telephone No. 91 3493277 |

Form PCT/ISA/210 (second sheet) (January 2015)